# EUROPEAN PATENT SPECIFICATION

(11) **EP 1 463 961 B1**
(45) Date of publication and mention of the grant of the patent: **03.11.2010**
(21) Application number: 03703715.7
(22) Date of filing: 08.01.2003
(51) Int. Cl.: G01T 1/161, A61B 19/00, A61B 6/04

(54) **OPEN-ACCESS EMISSION TOMOGRAPHY SCANNER**
EMISSIONSTOMOGRAFIESCANNER MIT OFFENEM ZUGANG
SCANNER DE TOMOGRAPHIE PAR EMISSION LIBRE ACCES

(30) Priority: 08.01.2002 US 346792 P
(43) Date of publication of application: 06.10.2004
(73) Proprietor: Naviscan, Inc., Rockville MD 20855 (US)
(72) Inventor: WEINBERG, Irving, Bethesda, MD 20817 (US); BEYLIN, David, Germantown, MD 20874 (US); STEPANOV, Pavel, Gaithersburg, MD 20878 (US); YARNALL, Steve, Poway, CA 92604 (US); ZAWARZIN, Valera, Newton, MA 02460 (US)
(74) Representative: Körber, Martin Hans
(86) International application number: PCT/US2003/000368
(87) International publication number: WO 2003/058275

(56) References cited:
- WO-A-95/21582
- WO-A-99/09431
- US-A- 5 519 221

## Description

### Field of the Invention

The present invention relates to an apparatus and a method for implementing emission tomography of a body part, and more particularly an apparatus and a method for implementing emission tomography of a body part with open access for medical or surgical interventions, such as a biopsy or minimally invasive therapy.

### Description of the Related Art

Nuclear medicine imaging measures the distribution of injected radiotracers to describe biochemistry and/or physiology of a body part *in vivo*. The uses of compression and/or immobilization of a body part during the act of physiological image acquisition have been previously described in several patents, including U.S. Patent No. 5,252,830, U.S. Patent No. 5,323,006, and U.S. Patent No. 5,519,221. When the image acquisition is performed using a radiotracer emitting a single gamma-ray, and the body part is a breast that is compressed or immobilized by a body part holder or holders, and image acquisition is performed with one or more gamma-ray detector modules in close proximity to the breast, the procedure can be described as compression scintimammography. When the image acquisition is performed using a radiotracer emitting two coincident gamma-rays (i.e., as in positron annihilation), and the body part is a breast that is compressed or immobilized by a body part holder or holders, and image acquisition is performed with one or more gamma-ray detector modules in close proximity to the breast, the procedure can be described as positron emission mammography.

It is preferable to sample the body part, or remove tissue from the body part, without releasing the body part from its compressed or immobilized position. This preference is due to the loss in spatial accuracy that may occur if the body part moves from the time that a target is selected on the basis of images, to the time that the target is sampled or removed. In order to perform an intervention, it is preferable to gain access to the body part through the body part holder, via one or more apertures in the body part holder.

Prior to the current invention, gamma-ray detector heads were generally stationary with respect to the body part holder during the process of gamma-ray detection, and it was necessary to remove the gamma-ray detector modules from the body part holders in order to provide access to the aperture when interventions were contemplated. For example, see R. Raylman et al., "Positron Emission Mammography-Guided Breast Biopsy", J. Nucl. Med. 2001, Vol. 42, pp. 960-966. Thus, the present inventors have observed that it would be advantageous to provide a capability to move the gamma ray detector heads during the detection process and thereby obviate the need to remove one or more of the detector modules from the body part holders during interventions.

Conventional body part holders on mammography equipment have large (e.g., 5 cm x 5 cm) open apertures that allow a portion of the breast to protrude during compression. This unrestrained portion of the breast may move during physical interventions such as needle insertions. Tissue movement can affect the ability to accurately target suspicious tissue for biopsy or treatment. Reducing the size of the aperture could better stabilize the tissue; however, interventions become more difficult since the accessible area is reduced. Accordingly, the present inventors have recognized a need for a means to stabilize the tissue which is being accessed through the aperture without substantially reducing access to the tissue.

### SUMMARY OF THE INVENTION

In one aspect, the invention provides a PET diagnostic system as defined in claim 1, for imaging a functional abnormality in a body part into which a radiotracer has been injected into the body part. The system includes at least one body part holder and at least one gamma ray detector module. Each body part holder immobilizes and compresses the body part. Each body part holder includes an aperture. The aperture provides access to the body part for performing a medical intervention. Each gamma ray detector module is movable within mechanical stages which are affixed to the holder with respect to the at least one body part holder. The gamma ray detector module obtains data by detecting gamma rays emitted by the injected radiotracer, and the system uses the obtained data to provide an image of the functional abnormality.

The body part holder may include at least one safety door which is hingedly affixed to the aperture of the body part holder. The safety door may prevent the body part from protruding through the aperture whenever the safety door is in a closed position. The safety door may prevent the gamma ray detector module from moving past the aperture whenever the safety door is in an open position. The safety door may be movable and removable with respect to the aperture. The safety door may include at least one open slot such that a portion of the body part to which access is provided by the aperture can be varied by movement or removal of the safety door, and such that the at least one safety door substantially stabilizes the portion of the body part to which access is provided by the aperture, and such that the access is not substantially reduced by the safety door. The safety door may also include a plurality of bars. The bars may displace the body part in order to prevent pinching of the body part during movement or removal of the safety door.

The body part holder may include a first safety door and a second safety door. The first safety door may be configured to open and close in a horizontal orientation with respect to the body part holder, and the second safety door may be configured to open and close in a vertical orientation with respect to the body part holder. The body part holder may also include a plurality of holes to allow the body part to be marked or to be accessed for sampling or for later identification. The body part holder may be fabricated using transparent material. The radiotracer may comprise a single-photon-emitter or a position emitter. The body part holder may include a fiduciary marker which is configured to facilitate the use of an additional medical device in conjunction with the system to improve the quality of the imaging of the functional abnormality. The additional medical device may comprise a stereotactic x-ray mammography device.

The gamma ray detector module may include a printed circuit board. The printed circuit board may include scintillators, light guides, and position-sensitive photomultipliers. The printed circuit board may generate a signal, the signal including the obtained data, and the signal may be transmitted to a processor. The processor may use the transmitted signal to produce the image of the functional abnormality. The scintillators may comprise a plurality of arrays of lutetium-based crystals. The processor may be configured to receive x-ray data and to use the received x-ray data to improve the quality of the produced image of the functional abnormality. The processor may be configured to produce the image of the functional abnormality by using a maximum-likelihood iterative reconstruction algorithm. The processor may be configured to produce an anatomic image from the received x-ray data. The processor may include a graphical user interface configured to simultaneously display the anatomic image and the image of the functional abnormality. The anatomic image may be a projection of the x-ray, or a combination of projections. Typical x-ray projections used in mammography include a cranio-caudal projection, a medio-lateral projection, and +15 and -15 degree obliques to these projections. The image of the functional abnormality may be a typical x-ray mammography projection or a combination of projections, or oblique or orthogonal views to these projections, or a processed projection or combination of projections (e.g., maximal intensity projection image, in which the maximal value for each pixel along a projection is displayed). When a user indicates a specific location within the image of the functional abnormality, the graphical user interface may indicate a corresponding location within the anatomic image. When a user indicates a specific location within the anatomic image, the graphical user interface may indicate a corresponding location within the image of the functional abnormality. The system may include at least one optoelectronic element configured to determine a position of the gamma ray detector module.

A method for imaging a functional abnormality in a body part includes the steps of injecting a radiotracer into the body part; using an apparatus to immobilize and compress the body part, detecting gamma rays being emitted by the radiotracer by moving at least one gamma ray detector across the aperture; and using the detected gamma rays to produce an image of the functional abnormality. The apparatus includes an aperture that provides access to a portion of the body part. The method may also include the step of implementing a safety mechanism to protect the body part from the moving gamma ray detector. The safety mechanism may be physically affixed to the apparatus at the aperture. The step of implementing a safety mechanism may include the step of closing at least one safety door that is hingedly affixed to the apparatus. The safety mechanism may be movable and removable with respect to the aperture. The method may also include the step of moving and/or removing the safety mechanism in order to allow access to a variety of portions of the body part. The safety mechanism may be configured to prevent pinching of the body part during movement or removal of the safety mechanism.

The method may also include the steps of obtaining an additional image of the body part by using an additional medical device and displaying data from the image of the functional abnormality in conjunction with a display of data from the obtained additional image. The additional medical device may comprise an x-ray device. The method may also include the step of using a fiduciary marker to facilitate the use of the x-ray device. When a user indicates a specific location within the image of the functional abnormality, the method may include the step of indicating a corresponding location within the obtained additional image. When the user indicates a specific location within the obtained additional image, the method may include the step of indicating a corresponding location within the image of the functional abnormality. The method may include the step of using at least one optoelectronic element to continuously determine a position of the gamma ray detector. The radiotracer may comprise a single-photon emitter or a positron emitter.

### BRIEF DESCRIPTION OF THE DRAWINGS

Figure 1 shows a side view of an open-access emission tomography scanner apparatus according to a preferred embodiment of the present invention.

Figure 2 shows a first perspective view of the open-access emission tomography scanner apparatus of Figure 1.

Figure 3 shows a second perspective view of the open-access emission tomography scanner apparatus of Figure 1.

Figure 4 shows a series of four configurations of the safety doors of the scanner apparatus of Figure 1.

Figure 5 shows a flowchart illustrating a method of implementing the apparatus of Figure 1.

### DETAILED DESCRIPTION OF THE INVENTION

In a preferred embodiment of the present invention, the gamma-ray detector modules move within mechanical stages that are affixed to the holder or holders during the process of gamma-ray detection, so that access is provided to perform interventions on the body part without the need to remove the mechanical stages from the holder(s). Besides intervention, the aperture can also provide access to diagnostic tools that are dependent on close proximity or contact to the body part. Examples of such diagnostic tools include ultrasound, optical tomography, gamma or beta probes, and coils for magnetic resonance imaging. The use of moving gamma-ray detector modules affords economy and flexibility as compared to a stationary gamma-ray detector module, because a wider area can be surveyed with the moving gamma-ray detector modules than with the stationary modules, assuming that the gamma-ray acquisition process is efficient enough that a sufficient quantity of count statistics can be accumulated by the traveling gamma-ray detector modules. Fortunately, the gamma-ray collection efficiency of positron emission mammography is so high that very good quality images can be obtained under such conditions. For a further discussion of the gamma-ray collection efficiency of positron emission mammography, see C. Thompson et al., "Feasibility Study for Positron Emission Mammography", Med. Phys. 1994, Vol. 21, pp. 529-538.

In order to provide a user with feedback as to the nature and quality of the image of the body part during the process of gamma-ray acquisition, it is helpful to be able to perform image reconstruction in parts, so that as a new section of the body part is being surveyed, data from the body part which was previously surveyed is being backprojected, reconstructed, and/or displayed to the user. Because the gamma-ray detectors are moving with respect to the immobilized body part in a preferred embodiment of the invention, it is preferable to insure that no portion of the body part extends out of the aperture during travel of the detectors past the aperture, lest said portion be pinched or otherwise damaged by a gamma-ray detector module. Accordingly, in a preferred embodiment of the invention, a safety door or other body-part trapping apparatus is affixed to the aperture so that the body part does not extend past the body-part holder into the path of the traveling gamma-ray detector module(s).

In a preferred embodiment of the present invention, a fail-safe mechanism is provided in order that if the safety door or other body-part trapping apparatus does not effectively contain the body part out of the path of the traveling gamma-ray detector module(s), the gamma-ray detector module(s) are barred from traveling past the aperture. In a preferred embodiment, the fail-safe apparatus is implemented via mechanical means by having the door resist motion of the traveling gamma-ray detector module(s) when the safety door is not closed. In addition, a preferred embodiment of the present invention provides movable safety doors with open slots, which stabilize the tissue which is being accessed through the aperture, without substantially reducing access to the tissue.

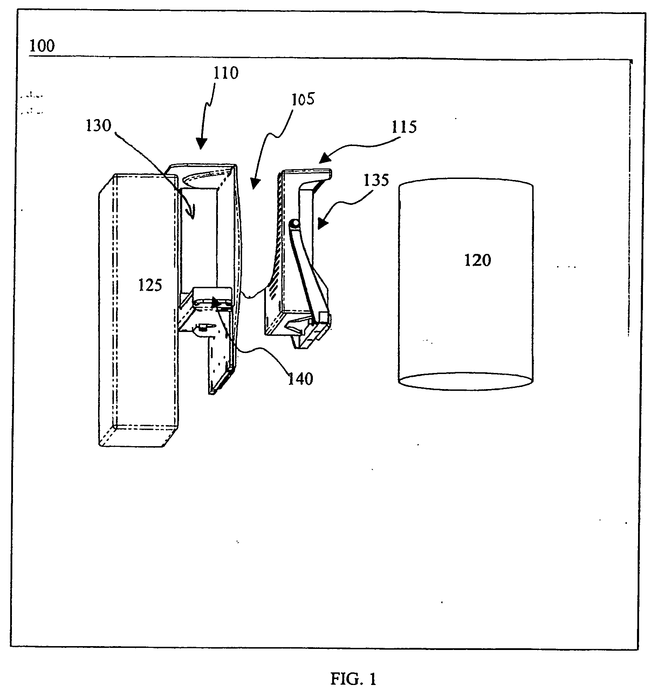

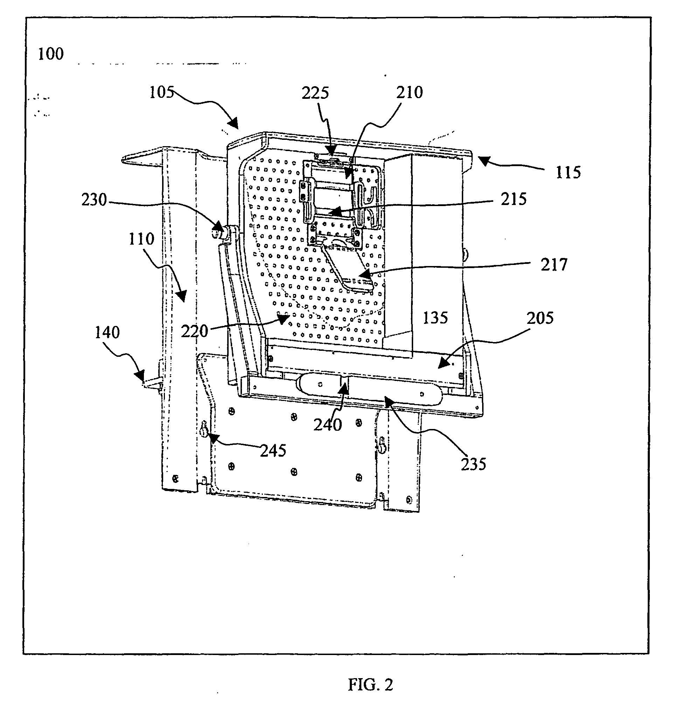

The present invention covers only embodiments relating to position emission tomography (PET). Referring to Figure 1, a side view of an apparatus 100 according to one embodiment of the present invention is shown. In this embodiment, a patient lies above the apparatus 100, with the body part 105 to be examined (e.g., a breast) pendant through an aperture in a table (table not shown in Fig. 1). The body part 105 is immobilized and/or compressed by body part holders 110 and 115, said body part holders being relatively transparent to x-ray irradiation, and which are disposed on two sides of the body part 105. An x-ray emitter 120 is able to project x-rays through the body part 105, and through the body part holders 110 and 115 onto an x-ray sensor 125. In a preferred embodiment, the body part holders 110 and 115 are transparent to x-rays; however, the body part holders may be relatively transparent to other types of radiation or sources of signal providing anatomic information about the body part, such as, for example, MRI signals, ultrasound signals, infrared signals, or visible light signals. The tops of the body part holders 110 and 115 support the patient's body and keep the patient's body from directly contacting gamma-ray detector modules 130 and 135. In one embodiment, the modules 130 and 135 detect gamma rays emitted by the body part. However, the apparatus 100 may include detector modules that are sensitive to any signal (including gamma rays or any other signal) emitted by the body part which contains physiological information about the body part. The gamma-ray detector modules 130 and 135 are supported and moved by mechanical stages that are affixed to the body part holders 110 and 115. In Figure 1, one of the mechanical stages 140 is shown, while the other stage is obscured by body part holder 115. Referring also to Figure 2, the obscured mechanical stage 205 is shown in a perspective view of the apparatus 100. Body part holders 110 and 115 also have means of coupling to the table.

Referring again to Figure 2, body part 105 (again, typically a breast) is shown immobilized and/or compressed by body part holders 110 and 115. Body part holder 115 has an aperture 210 and safety doors 215 and 217, as well as multiple holes 220. The holes 220 in body part holder 115 can admit marking pen tips or other thin devices in order to mark the patient's skin if necessary. Preferably, body part holder 115 is fabricated from transparent material in order to better enable sight of the body part 105. Aperture 210 is shown with horizontal safety door 215 closed and with vertical safety door 217 open. Horizontal safety door 215 has holes that can admit needles or other interventional devices. Horizontal safety door 215 can swing open horizontally, and can also move up and down vertically, so that if the bars of the horizontal safety door 215 block the access of the needles, the bars can be repositioned. The bars of the horizontal safety door 215 displace the body part 105 away from the aperture 210 to keep the breast from being pinched as the horizontal safety door 215 is opened, swung on its hinge, or closed. The horizontal safety door 215 can be repositioned to cover different portions of the body part 105 by swinging the horizontal safety door 215 open on its hinge, or by replacing the horizontal safety door 215 in a different position, or by removing the horizontal safety door 215 entirely. This repositioning capability allows the body part 105 to be restrained and immobilized, while providing access to the area of the entire aperture 210. The open vertical safety door 217 does not permit travel of gamma-ray detector module 135. Thus, the safety doors 215 and 217 provide the following functions: (1) they restrain the body part 105 from protruding through the aperture; (2) they immobilize the body part 105 for improved accuracy during procedures; and (3) they act as safety measures by blocking travel of the detector modules 130 and 135 while safety door 217 is not closed.

Referring again to Figure 2, body part holder 115 includes a metal strip 225 which has a hole in its center. The x-ray projection of this hole can be used as a fiduciary marker in the following way: A compression paddle (body part holder) that is supplied by a manufacturer of a stereotactic x-ray mammography unit has a similar metal strip and hole. After the stereotactic x-ray mammography unit is calibrated with this native compression paddle, the user places a pin (said pin is also supplied by the manufacturer, and said pin is affixed to the table by the user during the calibration process) in the hole. The user then removes the native compression paddle and replaces the native compression paddle with body part holder 115, aligning the hole in metal strip 225 with the same pin, by making fine adjustments with screw 230 and other adjustment points. The hole in metal strip 225 can be used by the stereotactic x-ray mammography unit to calibrate software needed to perform biopsies through aperture 210. In a preferred embodiment, gamma-ray detector module 135 includes scintillators, compact light guides, and compact position-sensitive photomultipliers on a printed circuit board that carries high voltage levels (which are needed to operate the photomultipliers) and a signal, wherein said signal is conveyed to a trigger and signal processing system, computer, and display system, such as that described in U.S. Patents Nos. 5,252,830; 5,323,006; and 5,519,221. Gamma-ray detector module 135 is conveyed by mechanical stage 205. Gamma-ray detector module 130 (not visible on Figure 2), which travels on body part holder 110, is conveyed by mechanical stage 140. Body part holder 110 fastens to the table of the stereotactic mammography unit via dovetail 235, which has a detent 240 to aid in alignment. Body part holder 110 attaches to the stereotactic mammography unit via screws through holes 245, and can have its position adjusted with respect to the stereotactic mammography unit by appropriate placement of screws through said holes 245.

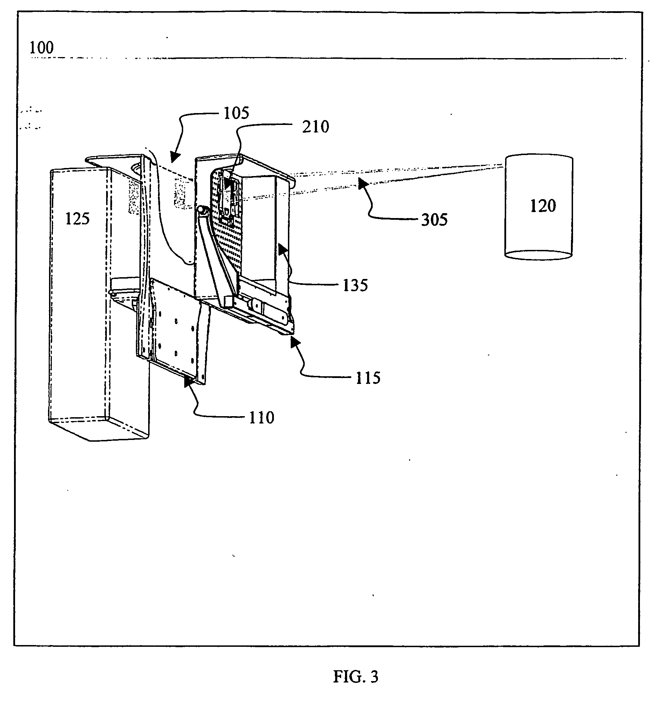

Referring to Figure 3, a second perspective view of the system configuration of the apparatus 100 is shown. The x-ray sensor 125 is illuminated by an x-ray beam 305 from x-ray source 120. X-ray beam 305 traverses body part holders 110 and 115, as well as body part 105 and aperture 210. Gamma-ray detector module 135 is visible in Figure 3 while the other gamma-ray detector module 130 is not visible in this view. The gamma-ray detector modules 130 and 135 travel under computer control upon the mechanical stages 140 and 205 respectively. The purpose of this travel is to provide maximum flexibility so that the gamma-ray detector modules 130 and 135 can be swept across either the entire body part 105 (e.g., in order to acquire a full-breast image), or across the central portion of the body part 105 (e.g., in order to correlate with digital spot mammograms). When not acquiring gamma-ray data, the gamma-ray detector modules 130 and 135 are parked on one side of the mechanical stages 140 and 205 in order to provide access to the central field-of-view for biopsy or digital spot mammography x-rays. The gamma-ray detector modules 130 and 135 can be moved independently, so that they can move in the same direction across the body part 105, or they can be moved in opposite directions. Optoelectronic detector elements confirm location of the gamma-ray detector modules 130 and 135.

In a preferred embodiment of the apparatus 100 known as the "PEM-2400" (produced by PEM Technologies, Inc. of Rockville, Maryland, the assignee of the subject application), each of the gamma-ray detector modules 130 and 135 includes twelve arrays of 13x13 lutetium-based crystals. Each crystal measures 2 mm x 2 mm x 10 mm. The arrays are affixed to Hamamatsu R-8520-C12 position-sensitive photomultipliers ("PS-PMTs") with structured light guides to minimize gaps between arrays. In an exemplary process used for examining a breast, the PS-PMTs are read out with a multiplexing strategy in which events between PS-PMTs that are not adjacent to one another do not combine in the charge readout. External detector shielding is employed (i.e., 1 mm tungsten on five sides of each gamma-ray detector module, and 1.5 mm aluminum on the side facing the breast) such that the shielding minimally interferes with close access to the patient's chest wall. Last dynode signals that are ganged within each gamma-ray detector module are sent to a trigger board for coincidence testing. Triggering is accomplished with a two-step logic, in which a lower threshold is set to trigger on the first few electrons just above the noise level, and a higher threshold determines the desired energy threshold. The height of the gamma-ray detector modules is set so that the gamma-ray image's field-of-view overlaps the x-ray height by 2 mm. With this overlap, the entire x-ray field can be imaged with the PEM-2400. This is motivated by a desire to image as much of the posterior chest as possible. Considering that the degree of compression required for high sensitivity is not as demanding as for x-ray mammography, more of the posterior chest may be visible with the full-field PEM-2400 than with x-ray mammography, because of the ability to pull more of the breast into the table aperture and still maintain enough compression to get adequate gamma-ray images.

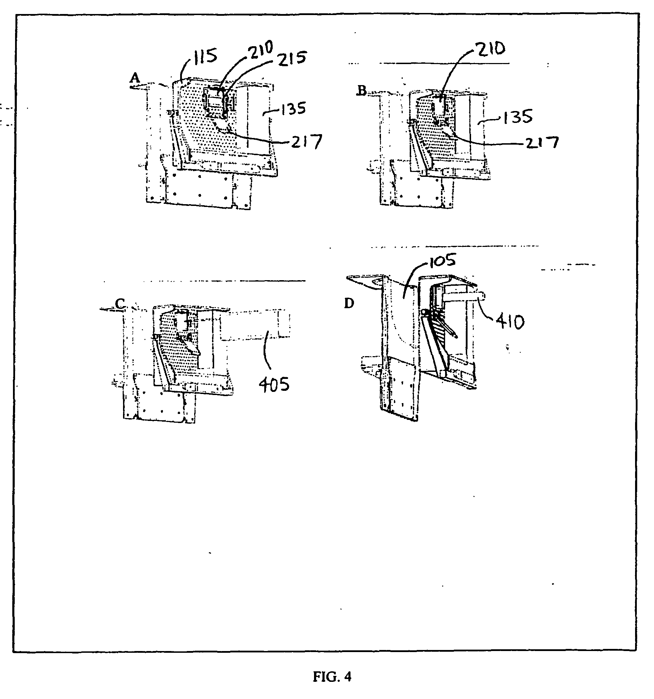

The use of moving gamma-ray detector modules that sweep past the breast imposes the need to address safety concerns, because without safeguards, the breast might protrude through an open biopsy aperture and be exposed to the moving detectors. As discussed above, this risk is mitigated by using hardware interlocks ("safety doors") that physically stop motion of the gamma-ray detector modules if the safety doors are not closed. Furthermore, the motor drive is set so that even minimal resistance will stop the gamma-ray detector modules. Referring to Figure 4, operation of the safety doors is shown as an interlock. Panel A shows body part holder 115 with horizontal gate 215 closed across aperture 210, and vertical gate 217 open, ready for biopsy or x-ray. The gamma-ray detector module 135 would not be able to move across aperture 210 because horizontal gate 215 would stop it. Panel B shows both gates 215 and 217 open, ready for biopsy or x-ray, with gamma-ray detector head 135 out of the way. Panel C shows both gates open, with biopsy gun 405 being used to place a needle through aperture 210. Panel D shows both gates open, with device 410 in close proximity to breast 105. Device 410 could be an ultrasound transducer or a gamma probe.

Optoelectronic elements are set along the tracks in the mechanical stages 140 and 205 which hold the respective gamma-ray detector modules 130 and 135 so that the position of the gamma-ray detector modules is known at all times, even when the system 100 has lost power and recovered. Ribbon cables are looped within the mechanical stages 140 and 205 so that the gamma-ray detector modules 130 and 135 can move freely from one side of the mechanical stages 140 and 205 to the other without impediment. Each of the gamma-ray detector modules 130 and 135 is under separate software control, although in routine operation both gamma-ray detector modules move in the same direction. Note that because the gamma-ray detector modules 130 and 135 are very thin (approximately 6 cm in thickness), it is possible to obtain straight and ±15-degree x-ray images without removing the body part holders or x-ray sensors from the stereotactic x-ray mammography camera. This feature is important, because as a practical matter, the x-ray sensor is quite heavy and delicate, and thus it would be onerous to ask a technologist to remove the x-ray sensor in routine cases. The body part holders 110 and 115 have identical x-ray transmission specifications as compared to the native stereotactic unit paddle.

Files of detected gamma-ray signals are created in list-mode, with a separate list mode file created for delayed coincidences to accomplish later random subtractions. Images are backprojected during image acquisition, as the positron emission tomography ("PET") detector heads sweep across the breast, in order to provide feedback to the operator that the study is going well. Calibration is performed once a day using a sheet source for calculating matrices to correct for energy and spatial nonlinearity and efficiency correction. The history of deviations from initial factory settings is recorded for later analysis. A second calibration is performed every day to check the spatial correlation between the x-ray and the positron emission mammography ("PEM") image.

Images are captured from the x-ray mammography camera via a frame-grabber operating from a monitor video signal from the stereotactic mammography computer. In this manner, there is no interference with any of the software functions in the native stereotactic x-ray mammography system.

Reconstruction is performed using a maximum-likelihood iterative reconstruction. In one embodiment, a graphical user interface (GUI) is implemented in Linux (RedHat 7.2 distribution) using C, C++, GTK+, and XML code which was inspired by the Amide package created by Andy Loening at UCLA. One software package in use with the PEM-2400 includes analysis tools such as region-of-interest calculation and electronic virtual calipers. Using the values established by the user in the x-ray correlation calibration, the user can click on the x-ray and find the corresponding location on the appropriate PET slice, and vice versa. It is noted that for the x-ray, image types may include a cranio-caudal projection, a mediolateral projection, or a 15-degree oblique projection, among other possible projections. For PET, image types may include, among others, a cranio-caudal projection, a projection that is orthogonal to the cranio-caudal, a mediolateral projection, a projection that is orthogonal to the mediolateral, and 15-degree oblique projections.

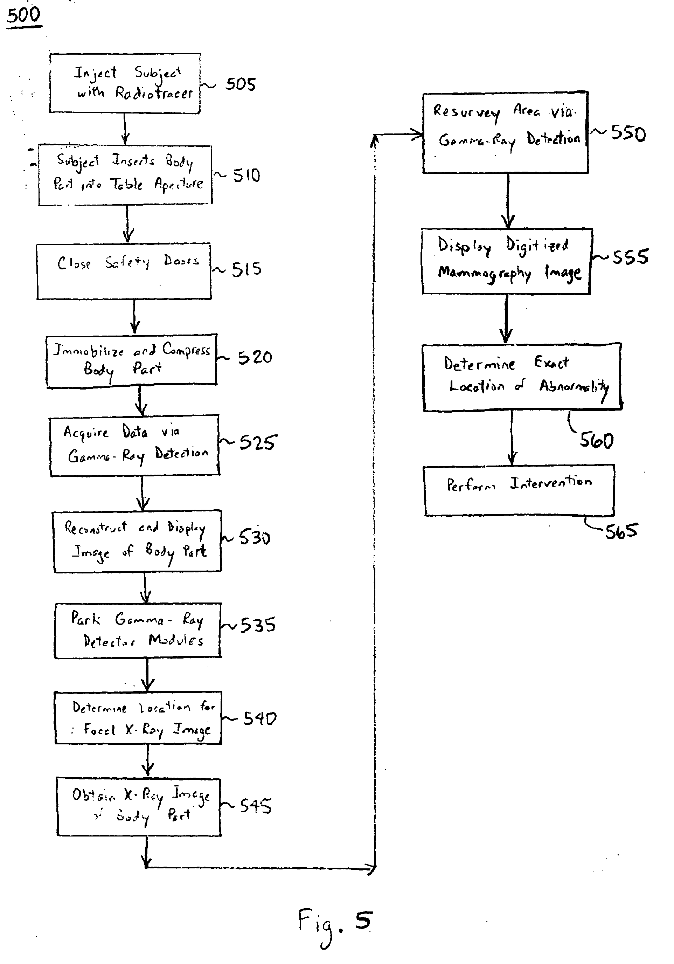

Referring to Figure 5, a flowchart illustrates an exemplary method according to a preferred embodiment of the invention. At step 505, a human subject is injected with 10 milliCuries of 2-fluorodeoxyglucose intravenously, and the subject is instructed to rest quietly prior to imaging with the PEM-2400 scanner. At step 510, the subject lies down in prone position upon a stereotactic mammography table, and inserts her breast into a wide table aperture as she would typically do for a stereotactic biopsy. At step 515, the safety doors are closed on the body part holder, so that portions of the breast will not protrude through the aperture when compression is applied. At step 520, the body part holders then apply compression to opposite sides of the breast. These body part holders have similar x-ray transmission and biopsy capability properties as the compression paddle or paddles that would normally be used with the stereotactic mammography table. At step 525, the user operates the PEM-2400 gamma-ray detector modules with computer control, and starts data acquisition, which is implemented by using the PEM-2400 gamma-ray detector modules to collect data as the modules sweep across the breast. At step 530, images of portions of the breast that have already been passed over are reconstructed and displayed during travel by the gamma-ray detector modules over other parts of the breast. When this full-breast image has been completed, at step 535, the PEM-2400 gamma-ray detector modules are parked on the side of the trays that is opposite from the human user (i.e., the operator of the PEM-2400). At step 540, the human user can inspect the PEM-2400 images and decide where a focal x-ray image or additional physiological image (e.g., an image obtained using positron emission mammography) should be performed. Holes in the body part holder can accommodate marking pen tips to allow the user to draw markings on the surface of the breast to aid in repositioning, or to install locational wires for marking tumor locations. At step 545, x-rays can be obtained of the compressed breast by firing the x-ray source. Markings on the holders can be seen on the x-rays, enabling registration of the physiological images with the x-ray images. The x-rays form a digital image that is smaller than the aperture in the holder. At step 550, if necessary, the PEM-2400 gamma-ray detector modules can be moved under computer control to resurvey the area of the aperture. The PEM-2400 gamma-ray detector modules can be mounted on opposite (e.g., diagonally opposite) sides of the aperture in order to image radioactive needles during the intervention or to image remaining cancer tissue during an intervention. At step 555, the PEM-2400 digitizes the video monitor output of the digital spot x-ray mammography device and places this image on the PEM-2400 monitor, in order to facilitate cross-modality comparisons. At step 560, the user can click on a location in the functional image generated by the PEM-2400, and a corresponding marker is generated in the x-ray images (e.g., +15, -15, and straight views) to show the user where the functional abnormality is present on the anatomic image. Because the captured x-ray monitor images also show the location of the cursor that the user uses in order to specify locations for biopsy, the net effect is that the user can see how close the intended biopsy location (e.g., as suggested by the spot x-ray) is to the region of the functional abnormality. Finally, at step 565, the user performs an intervention, such as a biopsy of the abnormal area.

While the present invention has been described with respect to what is presently considered to be the preferred embodiment, it is to be understood that the invention is not limited to the disclosed embodiments. For example, it is to be understood that the invention is applicable to body parts other than the breast, body parts from non-human animals and parts of inanimate objects, using detection means that are sensitive to signals other than gamma rays, whether or not the signals arrive at the detectors in coincidence. Furthermore, although in the preferred embodiments, the body part holders are mounted on an x-ray mammography camera, the body part holders can apply compression while mounted on a stand-alone platform that does I not perform x-ray examination.

## Claims

1. A position emission tomography (PET) diagnostic system (100) for imaging a functional abnormality in a body part, a radiotracer having been injected into the body part, the system comprising:
at least one body part holder (110; 115) configured to immobilize and compress the body part, wherein the at least one body part holder includes an aperture (210), and wherein the aperture is configured to provide access to the body part for performing a medical intervention; and
at least one gamma ray detector module (135),
the system being **characterized by** the at least one gamma ray detector module (135) being movable with respect to the at least one body part holder such that a wider area can be surveyed with the movable at least one gamma ray detector than could be surveyed with a stationary gamma ray detector;
wherein the at least one gamma ray detector module (135) is configured to obtain data by detecting gamma rays while moving within mechanical stages affixed to the holder with respect to the at least one body part holder (110; 115), the detected gamma rays being emitted by the injected radiotracer, and the system is configured to use the obtained data to provide an image of the functional abnormality and
wherein the at least one gamma ray detector comprises at least a first positron emission tomography (PET) detector and a second PET detector.

2. The diagnostic system (100) of claim 1, further **characterized by** the at least one body part holder including at least one safety door (215; 217), the at least one safety door being hingedly affixed to the aperture of the at least one body part holder (110; 115), and
the at least one safety door (215; 217) being configured to prevent the body part from protruding through the aperture whenever the at least one safety door is in a closed position and/or to prevent the at least one gamma ray detector module (135) from moving past the aperture whenever the at least one safety door is in an open position.

3. The diagnostic system (100) of claim 2, further **characterized by** the at least one safety door (215; 217) being configured to be movable and removable with respect to the aperture, and the at least one safety door including at least one open slot,
such that a portion of the body part to which access is provided by the aperture can be varied by movement or removal of the at least one safety door (215; 217), and such that the at least one safety door is configured to substantially stabilize the portion of the body part to which access is provided by the aperture, and such that the access is not substantially reduced by the at least one safety door.

4. The diagnostic system (100) of claim 3, further **characterized by** the at least one safety door (215; 217) further including a plurality of bars, the bars being configured to displace the body part in order to prevent pinching of the body part during movement or removal of the at least one safety door.

5. The diagnostic system (100) of claim 2, further **characterized by** the at least one body part holder (110; 115) including a first safety door (215) and a second safety door (217), the first safety door being configured to open and close in a horizontal orientation with respect to the at least one body part holder, and the second safety door being configured to open and close in a vertical orientation with respect to the at least one body part holder.

6. The diagnostic system (100) of claim 1, further **characterized by** the at least one body part holder including a plurality of holes configured to allow the body part to be marked.

7. The diagnostic system (100) of claim 1, further **characterized by** the at least one body part holder including a plurality of holes configured to allow a portion of the body part to be accessed for sampling or for later identification.

8. The diagnostic system (100) of claim 1, further **characterized by** the at least one body part holder being fabricated using transparent material.

9. The diagnostic system (100) of claim 1, further **characterized by** the radiotracer including a single-photon emitter.

10. The diagnostic system (100) of claim 1, further **characterized by** the radiotracer including a positron emitter.

11. The diagnostic system (100) of claim 1, further **characterized by** the at least one body part holder including a fiduciary marker, wherein the fiduciary marker is configured to facilitate the use of an additional medical device in conjunction with the system to improve the quality of the imaging of the functional abnormality.

12. The diagnostic system (100) of claim 11, further **characterized by** the additional medical device comprising a stereotactic x-ray mammography device.

13. The diagnostic system (100) of claim 1, further **characterized by** the at least one gamma ray detector module (135) including a printed circuit board, the printed circuit board comprising
a plurality of scintillators;
a plurality of light guides; and
a plurality of position-sensitive photomultipliers,
wherein the printed circuit board is configured to generate a signal, the signal including the obtained data, and to transmit the signal to a processor, and the processor is configured to use the transmitted signal to produce the image of the functional abnormality.

14. The diagnostic system (100) of claim 13, further **characterized by** the plurality of scintillators comprising a plurality of arrays of crystals, wherein the crystals include lutetium.

15. The diagnostic system (100) of claim 13, further **characterized by** the processor being further configured to receive x-ray data and to use the received x-ray data to produce an anatomic image and to display the anatomic image in conjunction with a display of the image of the functional abnormality.

16. The diagnostic system (100) of claim 15, further **characterized by** the anatomic image comprising a projection selected from the group consisting of a cranio-caudal projection, a medio-lateral projection, a +15-degree oblique projection, a -15-degree oblique projection, and a combination of two or more of said projections.

17. The diagnostic system (100) of claim 15, further **characterized by** the image of the functional abnormality comprising a projection selected from the group consisting of a cranio-caudal projection, a projection that is orthogonal to the cranio-caudal projection, a medio-lateral projection, a projection that is orthogonal to the medio-lateral projection, a +15-degree oblique projection, a -15-degree oblique projection, and a combination or two or more of said projections.

18. The diagnostic system (100) of claim 15, further **characterized by** the processor being further configured to produce the image of the functional abnormality by using a maximum-likelihood iterative reconstruction algorithm.

19. The diagnostic system (100) of claim 15, further **characterized by** the processor including a graphical user interface configured to simultaneously display the anatomic image and the image of the functional abnormality,
wherein, when a user indicates a specific location within the image of the functional abnormality, the graphical user interface is configured to indicate a corresponding location within the anatomic image, and when a user indicates a specific location within the anatomic image, the graphical user interface is configured to indicate a corresponding location within the image of the functional abnormality.

20. The diagnostic system (100) of claim 15, further **characterized by** the processor including a graphical user interface configured to simultaneously display the anatomic image and the image of the functional abnormality,
wherein, when a user indicates a specific location within the image of the functional abnormality, the graphical user interface is configured to indicate a corresponding location within the anatomic image.

21. The diagnostic system (100) of claim 15, further **characterized by** the processor including a graphical user interface configured to simultaneously display the anatomic image and the image of the functional abnormality,
wherein, when a user indicates a specific location within the anatomic image, the graphical user interface is configured to indicate a corresponding location within the image of the functional abnormality.

22. The diagnostic system (100) of claim 1, further **characterized by** the system further comprising at least one optoelectronic element configured to determine a position of the at least one gamma ray detector module (135).

## Patentansprüche

1. Positron-Emission-Tomographie (PET) Diagnosesystem (100) zum Abbilden einer funktionalen Anomalie in einem Körperteil, wobei ein Radiotracer in das Körperteil eingeführt wurde, wobei das System aufweist
wenigstens einen Körperteil-Halter (110; 115), der dazu ausgelegt ist, das Körperteil stillzulegen und zusammenzudrücken, wobei der wenigstens eine Körperteil-Halter eine Öffnung (210) aufweist, und wobei die Öffnung dazu ausgelegt ist, einen Zugang zu dem Körperteil zur Durchführung eines medizinischen Eingriffs zu gewährleisten; und
wenigstens ein Gammastrahlen-Detektormodul (135),
wobei das System **dadurch gekennzeichnet ist, dass** das wenigstens eine Gammastrahlen-Detektormodul (135) bezüglich des wenigstens einen Körperteil-Halters bewegbar ist, so dass ein größeres Gebiet mit dem bewegbaren wenigstens einen Gammastrahlen-Detektormodul untersucht werden kann, als mit einem feststehenden Gammastrahlen-Detektormodul untersucht werden könnte;
wobei das wenigstens eine Gammastrahlen-Detektormodul (135) dazu ausgelegt ist, Daten durch Erfassen von Gammastrahlen zu gewinnen, während einer Bewegung innerhalb mechanischer Stufen, die an dem Halter befestigt sind, bezüglich des wenigstens einen Körperteil-Halters (110; 115), wobei die erfassten Gammastrahlen von dem eingeführten Radiotracer ausgesandt werden, und das System dazu ausgelegt ist, die gewonnenen Daten zu verwenden, um ein Bild der funktionalen Anomalie bereitzustellen und
wobei der wenigstens eine Gammastrahlen-Detektor wenigstens einen ersten Positron-Emission-Tomographie (PET) Detektor und einen zweiten PET-Detektor aufweist.

2. Diagnosesystem gemäß Anspruch 1, ferner **dadurch gekennzeichnet, dass** der wenigstens eine Körperteil-Halter wenigstens eine Sicherheitstür (215; 217) aufweist, wobei die wenigstens eine Sicherheitstür klappbar an der Öffnung des wenigstens einen Körperteil-Halters (110; 115) befestigt ist, und
die wenigstens eine Sicherheitstür (215; 217) dazu ausgelegt ist, den Körperteil daran zu hindern, durch die Öffnung zu ragen, wenn die wenigstens eine Sicherheitstür in einer geschlossenen Stellung ist und/oder das wenigstens eine Gammastrahlen-Detektormodul (135) daran zu hindern, sich an der Öffnung vorbei zu bewegen, wenn die wenigstens eine Sicherheitstür in einer geöffneten Stellung ist.

3. Diagnosesystem (100) gemäß Anspruch 2, ferner **dadurch gekennzeichnet, dass** die wenigstens eine Sicherheitstür (215; 217) dazu ausgelegt ist, bewegbar und entfernbar bezüglich der Öffnung zu sein, und die wenigstens eine Sicherheitstür wenigstens einen offenen Spalt enthält,
so dass ein Abschnitt des Körperteils, zu dem der Zugang durch die Öffnung gewährleistet wird, durch Bewegung oder Entfernung der wenigstens einen Sicherheitstür (215; 217) verändert werden kann, und so dass die wenigstens eine Sicherheitstür dazu ausgelegt ist, den Abschnitt der Körperteils, zu dem der Zugang durch die Öffnung gewährleistet wird, wesentlich zu stabilisieren, und so dass der Zugang durch die wenigstens eine Sicherheitstür nicht wesentlich verringert wird.

4. Diagnosesystem (100) gemäß Anspruch 3, ferner **dadurch gekennzeichnet, dass** die wenigstens eine Sicherheitstür (215; 217) ferner mehrere Stangen enthält, wobei die Stangen dazu ausgelegt sind, den Körperteil zu versetzen, um ein Einklemmen des Körperteils während der Bewegung oder Entfernung der wenigstens einen Sicherheitstür zu verhindern.

5. Diagnosesystem (100) gemäß Anspruch 2, ferner **dadurch gekennzeichnet, dass** der wenigstens eine Körperteil-Halter (110; 115) eine erste Sicherheitstür (215) und eine zweite Sicherheitstür (217) enthält, wobei die erste Sicherheitstür dazu ausgelegt ist, sich in einer horizontalen Ausrichtung bezüglich des wenigstens einen Körperteil-Halters zu bewegen und zu schließen, und die zweite Sicherheitstür dazu ausgelegt ist, sich in einer vertikalen Ausrichtung bezüglich des wenigstens einen Körperteil-Halters zu bewegen und zu schließen.

6. Diagnosesystem (100) gemäß Anspruch 1, ferner **dadurch gekennzeichnet, dass** der wenigstens eine Körperteil-Halter mehrere Löcher enthält, die dazu ausgelegt sind zu ermöglichen, dass der Körperteil markiert wird.

7. Diagnosesystem (100) gemäß Anspruch 1, ferner **dadurch gekennzeichnet, dass** der wenigstens eine Körperteil-Halter mehrere Löcher enthält, die dazu ausgelegt sind zu ermöglichen, auf einen Abschnitt des Körperteils zur Probenentnahme oder zur späteren Identifizierung zuzugreifen.

8. Diagnosesystem (100) gemäß Anspruch 1, ferner **dadurch gekennzeichnet, dass** der wenigstens eine Körperteil-Halter unter Benutzung eines transparenten Materials hergestellt wird.

9. Diagnosesystem (100) gemäß Anspruch 1, ferner **dadurch gekennzeichnet, dass** der Radiotracer einen Einzelphoton-Emitter enthält.

10. Diagnosesystem (100) gemäß Anspruch 1, ferner **dadurch gekennzeichnet, dass** der Radiotracer einen Positron-Emitter enthält.

11. Diagnosesystem (100) gemäß Anspruch 1, ferner **dadurch gekennzeichnet, dass** der wenigstens eine Körperteil-Halter eine Vertrauenskennzeichnung enthält, wobei die Vertrauenskennzeichnung dazu ausgelegt ist, die Benutzung einer zusätzlichen medizinischen Vorrichtung in Verbindung mit dem System zu erleichtern, um die Qualität des Abbildens der funktionalen Anomalie zu verbessern.

12. Diagnosesystem (100) gemäß Anspruch 11, ferner **dadurch gekennzeichnet, dass** die zusätzliche medizinische Vorrichtung eine stereotaktische Röntgenmammografie-Vorrichtung aufweist.

13. Diagnosesystem (100) gemäß Anspruch 1, ferner **dadurch gekennzeichnet, dass** das wenigstens eine Gammastrahlen-Detektormodul (135) eine Leiterplatte enthält, wobei die Leiterplatte aufweist
mehrere Szintillatoren;
mehrere Lichtleiter; und
mehrere lageempfindliche Photovervielfacher,
wobei die Leiterplatte dazu ausgelegt ist, ein Signal zu erzeugen, wobei das Signal die gewonnenen Daten enthält, und das Signal an einen Rechner zu übertragen, und der Rechner dazu ausgelegt ist, das übertragene Signal zu verwenden, um das Bild der funktionalen Anomalie zu erzeugen.

14. Diagnosesystem (100) gemäß Anspruch 13, ferner **dadurch gekennzeichnet, dass** die mehreren Szintillatoren mehrere Anordnungen von Kristallen aufweisen, wobei die Kristalle Lutetium enthalten.

15. Diagnosesystem (100) gemäß Anspruch 13, ferner **dadurch gekennzeichnet, dass** der Rechner ferner dazu ausgelegt ist, Röntgendaten zu empfangen und die empfangenen Röntgendaten zu verwenden, um ein Anatomiebild zu erzeugen, und um das Anatomiebild in Verbindung mit einer Anzeige des Bildes der funktionalen Anomalie darzustellen.

16. Diagnosesystem (100) gemäß Anspruch 15, ferner **dadurch gekennzeichnet, dass** das Anatomiebild eine Projektion aufweist, die aus der Gruppe ausgewählt ist, die aus einer cranio-caudalen Projektion, einer medio-lateralen Projektion, einer +15-Grad schiefen Projektion, einer -15-Grad schiefen Projektion und einer Kombination von zwei oder mehr der genannten Projektionen besteht.

17. Diagnosesystem (100) gemäß Anspruch 15, ferner **dadurch gekennzeichnet, dass** das Bild der funktionalen Anomalie eine Projektion aufweist, die aus der Gruppe ausgewählt ist, die aus einer cranio-caudalen Projektion, einer Projektion senkrecht zu der cranio-caudalen Projektion, einer medio-lateralen Projektion, einer Projektion senkrecht zu der medio-lateralen Projektion, einer +15-Grad schiefen Projektion, einer - 15-Grad schiefen Projektion und einer Kombination von zwei oder mehr der genannten Projektionen besteht.

18. Diagnosesystem (100) gemäß Anspruch 15, ferner **dadurch gekennzeichnet, dass** der Rechner ferner dazu ausgelegt ist, das Bild der funktionalen Anomalie durch Benutzen eines iterativen Maximalwahrscheinlichkeit-Rekonstruktionsalgorithmus zu erzeugen.

19. Diagnosesystem (100) gemäß Anspruch 15, ferner **dadurch gekennzeichnet, dass** der Rechner eine graphische Benutzerschnittstelle enthält, die dazu ausgelegt ist, das Anatomiebild und das Bild der funktionalen Anomalie zeitgleich darzustellen,
wobei, wenn ein Benutzer eine spezielle Stelle in dem Bild der funktionalen Anomalie angibt, die graphische Benutzerschnittstelle dazu ausgelegt ist, eine entsprechende Stelle in dem Anatomiebild anzugeben, und wenn ein Benutzer eine spezielle Stelle in dem Anatomiebild angibt, die graphische Benutzerschnittstelle dazu ausgelegt ist, eine entsprechende Stelle in dem Bild der funktionalen Anomalie anzugeben.

20. Diagnosesystem (100) gemäß Anspruch 15, ferner **dadurch gekennzeichnet, dass** der Rechner eine graphische Benutzerschnittstelle enthält, die dazu ausgelegt ist, das Anatomiebild und das Bild der funktionalen Anomalie zeitgleich darzustellen,
wobei, wenn ein Benutzer eine spezielle Stelle in dem Bild der funktionalen Anomalie angibt, die graphische Benutzerschnittstelle dazu ausgelegt ist, eine entsprechende Stelle in dem Anatomiebild anzugeben.

21. Diagnosesystem (100) gemäß Anspruch 15, ferner **dadurch gekennzeichnet, dass** der Rechner eine graphische Benutzerschnittstelle enthält, die dazu ausgelegt ist, das Anatomiebild und das Bild der funktionalen Anomalie zeitgleich darzustellen,
wobei, wenn ein Benutzer eine spezielle Stelle in dem Anatomiebild angibt, die graphische Benutzerschnittstelle dazu ausgelegt ist, eine entsprechende Stelle in dem Bild der funktionalen Anomalie anzugeben.

22. Diagnosesystem (100) gemäß Anspruch 1, ferner **dadurch gekennzeichnet, dass** das System ferner wenigstens ein optoelektronisches Element aufweist, das dazu ausgelegt ist, eine Position des wenigstens einen Gammastrahlen-Detektormoduls (135) zu bestimmen.

## Revendications

1. Système de diagnostic par tomographie par émission de positrons (PET) (100) pour l'imagerie d'une anomalie fonctionnelle dans une partie du corps, un radiotraceur ayant été injecté dans la partie du corps, le système comprenant :
au moins un support de partie de corps (110 ; 115) configuré pour immobiliser et comprimer la partie du corps, dans lequel ledit au moins un support de partie de corps comprend une ouverture (210), et dans lequel l'ouverture est configurée pour fournir l'accès à la partie du corps pour effectuer une intervention médicale ; et
au moins un module de détection de rayons gamma (135),
le système étant **caractérisé en ce que** ledit au moins un module de détection de rayons gamma (135) peut être déplacé par rapport audit au moins un support de partie de corps de sorte qu'une zone plus large puisse être surveillée avec ledit au moins un détecteur de rayons gamma mobile que celle qui pourrait être surveillée avec un détecteur de rayons gamma fixe ;
dans lequel ledit au moins un module de détection de rayons gamma (135) est configuré pour obtenir des données en détectant des rayons gamma tout en se déplaçant dans des étages mécaniques fixés au support par rapport audit au moins un support de partie de corps (110 ; 115), les rayons gamma détectés étant émis par le radiotraceur injecté, et le système est configuré pour utiliser les données obtenues pour fournir une image de l'anomalie fonctionnelle ; et
dans lequel ledit au moins un détecteur de rayons gamma comprend au moins un premier détecteur de tomographie par émission de positrons (PET) et un deuxième détecteur de PET.

2. Système de diagnostic (100) selon la revendication 1, **caractérisé en outre en ce que** ledit au moins un support de partie de corps comprend au moins une porte de sécurité (215 ; 217), ladite au moins une porte de sécurité étant fixée de manière articulée à l'ouverture dudit au moins un support de partie de corps (110 ; 115), et
ladite au moins une porte de sécurité (215 ; 217) est configurée pour empêcher la partie du corps de faire saillie à travers l'ouverture à chaque fois que ladite au moins une porte de sécurité est dans une position fermée et/ou pour empêcher ledit au moins un module de détection de rayons gamma (135) de se déplacer au-delà de l'ouverture à chaque fois que ladite au moins une porte de sécurité est dans une position ouverte.

3. Système de diagnostic (100) selon la revendication 2, **caractérisé en outre en ce que** ladite au moins une porte de sécurité (215 ; 217) est configurée pour être mobile et amovible par rapport à l'ouverture, et ladite au moins une porte de sécurité comprend au moins une fente ouverte,
de sorte qu'une portion de la partie du corps à laquelle l'accès est fourni par l'ouverture peut être modifiée par un déplacement ou un retrait de ladite au moins une porte de sécurité (215 ; 217), et de sorte que ladite au moins une porte de sécurité est configurée pour stabiliser sensiblement la portion de la partie du corps à laquelle l'accès est fourni par l'ouverture, et de sorte que l'accès n'est pas sensiblement réduit par ladite au moins une porte de sécurité.

4. Système de diagnostic (100) selon la revendication 3, **caractérisé en outre en ce que** ladite au moins une porte de sécurité (215 ; 217) comprend en outre une pluralité de barres, les barres étant configurées pour déplacer la partie du corps afin d'éviter de pincer la partie du corps pendant un déplacement ou un retrait de ladite au moins une porte de sécurité.

5. Système de diagnostic (100) selon la revendication 2, **caractérisé en outre en ce que** ledit au moins un support de partie de corps (110 ; 115) comprend une première porte de sécurité (215) et une deuxième porte de sécurité (217), la première porte de sécurité étant configurée pour s'ouvrir et se fermer dans une orientation horizontale par rapport audit au moins un support de partie de corps, et la deuxième porte de sécurité étant configurée pour s'ouvrir et se fermer dans une orientation verticale par rapport audit au moins un support de partie de corps.

6. Système de diagnostic (100) selon la revendication 1, **caractérisé en outre en ce que** ledit au moins un support de partie de corps comprend une pluralité de trous configurés pour permettre le marquage de la partie du corps.

7. Système de diagnostic (100) selon la revendication 1, **caractérisé en outre en ce que** ledit au moins un support de partie de corps comprend une pluralité de trous configurés pour permettre l'accès à une portion de la partie du corps pour un échantillonnage ou une identification ultérieure.

8. Système de diagnostic (100) selon la revendication 1, **caractérisé en outre en ce que** ledit au moins un support de partie de corps est fabriqué en utilisant un matériau transparent.

9. Système de diagnostic (100) selon la revendication 1, **caractérisé en outre en ce que** le radiotraceur comprend un émetteur de photons unique.

10. Système de diagnostic (100) selon la revendication 1, **caractérisé en outre en ce que** le radiotraceur comprend un émetteur de positrons.

11. Système de diagnostic (100) selon la revendication 1, **caractérisé en outre en ce que** ledit au moins un support de partie de corps comprend un marqueur de repère, dans lequel le marqueur de repère est configuré pour faciliter l'utilisation d'un dispositif médical supplémentaire conjointement avec le système pour améliorer la qualité de l'imagerie de l'anomalie fonctionnelle.

12. Système de diagnostic (100) selon la revendication 11, **caractérisé en outre en ce que** le dispositif médical supplémentaire comprend un dispositif de mammographie à rayons X stéréotactique.

13. Système de diagnostic (100) selon la revendication 1, **caractérisé en outre en ce que** ledit au moins un module de détection de rayons gamma (135) comprend une carte de circuit imprimé, la carte de circuit imprimé comprenant :
une pluralité de scintillateurs ;
une pluralité de guides de lumière ; et
une pluralité de photomultiplicateurs sensibles à la position,
dans lequel la carte de circuit imprimé est configurée pour générer un signal, le signal comprenant les données obtenues, et pour transmettre le signal à un processeur, et le processeur est configuré pour utiliser le signal transmis pour produire l'image de l'anomalie fonctionnelle.

14. Système de diagnostic (100) selon la revendication 13, **caractérisé en outre en ce que** la pluralité de scintillateurs comprend une pluralité de matrices de cristaux, dans lequel les cristaux comprennent du lutétium.

15. Système de diagnostic (100) selon la revendication 13, **caractérisé en outre en ce que** le processeur est en outre configuré pour recevoir des données de rayons X et pour utiliser les données de rayons X reçues pour produire une image anatomique et pour afficher l'image anatomique conjointement avec l'affichage de l'image de l'anomalie fonctionnelle.

16. système de diagnostic (100) selon la revendication 15, **caractérisé en outre en ce que** l'image anatomique comprend une projection sélectionnée dans le groupe consistant en une projection cranio-caudale, une projection médio-latérale, une projection oblique à +15 degrés, une projection oblique à -15 degrés, et une combinaison de deux ou plus desdites projections.

17. Système de diagnostic (100) selon la revendication 15, **caractérisé en outre en ce que** l'image de l'anomalie fonctionnelle comprend une projection sélectionnée dans le groupe consistant en une projection cranio-caudale, une projection qui est orthogonale à la projection cranio-caudale, une projection médio-latérale, une projection qui est orthogonale à la projection médio-latérale, une projection oblique à +15 degrés, une projection oblique à -15 degrés, et une combinaison de deux ou plus desdites projections.

18. Système de diagnostic (100) selon la revendication 15, **caractérisé en outre en ce que** le processeur est configuré pour produire l'image de l'anomalie fonctionnelle en utilisant un algorithme de reconstruction itératif à maximum de vraisemblance.

19. Système de diagnostic (100) selon la revendication 15, **caractérisé en outre en ce que** le processeur comprend une interface graphique utilisateur configurée pour afficher simultanément l'image anatomique et l'image de l'anomalie fonctionnelle,
dans lequel, lorsqu'un utilisateur indique un emplacement spécifique dans l'image de l'anomalie fonctionnelle, l'interface graphique utilisateur est configurée pour indiquer un emplacement correspondant dans l'image anatomique et, lorsqu'un utilisateur indique un emplacement spécifique dans l'image anatomique, l'interface graphique utilisateur est configurée pour indiquer un emplacement correspondant dans l'image de l'anomalie fonctionnelle.

20. Système de diagnostic (100) selon la revendication 15, **caractérisé en outre en ce que** le processeur comprend une interface graphique utilisateur configurée pour afficher simultanément l'image anatomique et l'image de l'anomalie fonctionnelle,
dans lequel, lorsqu'un utilisateur indique un emplacement spécifique dans l'image de l'anomalie fonctionnelle, l'interface graphique utilisateur est configurée pour indiquer un emplacement correspondant dans l'image anatomique.

21. Système de diagnostic (100) selon la revendication 15, **caractérisé en outre en ce que** le processeur comprend une interface graphique utilisateur configurée pour afficher simultanément l'image anatomique et l'image de l'anomalie fonctionnelle,
dans lequel, lorsqu'un utilisateur indique un emplacement spécifique dans l'image anatomique, l'interface graphique utilisateur est configurée pour indiquer un emplacement correspondant dans l'image de l'anomalie fonctionnelle.

22. Système de diagnostic (100) selon la revendication 1, **caractérisé en outre en ce que** le système comprend en outre au moins un élément optoélectronique configuré pour déterminer une position dudit au moins un module de détection de rayons gamma (135).
